# EUROPEAN PATENT APPLICATION

(11) **EP 4 696 700 A1**
(43) Date of publication of application: **18.02.2026**
(21) Application number: 24787970.3
(22) Date of filing: 03.04.2024
(51) Int. Cl.: C07K 7/06, A61K 38/00, A61P 25/04, A61P 17/04

(54) **LONG-ACTING K-OPIOID RECEPTOR AGONIST**

(30) Priority: 10.04.2023 CN 202310371590
(71) Applicant: Chengdu Aoda Biotechnology Co., Ltd, Chengdu, Sichuan 610041 (CN)
(72) Inventor: ZHOU, Shuliang, Chengdu, Sichuan 610041 (CN); WANG, Peng, Chengdu, Sichuan 610041 (CN); DENG, Lan, Chengdu, Sichuan 610041 (CN)
(74) Representative: PGA S.p.A., Milano, Succursale di Lugano
(86) International application number: PCT/CN2024/085854
(87) International publication number: WO 2024/212866

(57) **Abstract**

Provided is a long-acting K-opioid receptor agonist. The long-acting K-opioid receptor agonist is used for preparing a pharmaceutical composition for treating diseases, the pharmaceutical composition being used for relieving moderate to severe patient itchiness and postoperative analgesia.

## Description

This application claims the priority of Chinese Patent Application No. 202310371590.8, filed with the China National Intellectual Property Administration on April 10, 2023, and titled "LONG-ACTING K-OPIOID RECEPTOR AGONIST", which is hereby incorporated by reference in its entirety.

### FIELD

The present disclosure relates to a long-acting K-opioid receptor agonist and uses thereof.

### BACKGROUND

Opioid drugs exert physiological effects mainly through binding to three known classical opioid receptors µ, δ, and κ. These three receptors are all members of the G-protein-coupled receptor family, are mainly distributed in the central nervous system, and also exist in many peripheral tissues.

The most classical one of these drugs is morphine, which exerts an analgesic effect mainly through the µ-opioid receptor. Clinically common analgesic drugs also include other µ-opioid receptor drugs, such as traditional opioid drugs as represented by dihydromorphinone and fentanyl. However, long-term use of µ-opioid receptor drugs can lead to multiple side effects, such as tolerance, dependence, respiratory depression, effects on gastrointestinal motility, and the like, which not only increase treatment costs, but also affect recovery periods of patients. Due to their poor analgesic effects, some non-opioid injections, such as acetaminophen and non-steroidal anti-inflammatory drugs, have limited application ranges and dosages; furthermore, they also have certain side effects, for example, acetaminophen increases liver toxicity, and non-steroidal anti-inflammatory drugs can cause various gastrointestinal diseases.

Research has found that, using K opioid receptor agonists, the K-opioid receptor can be used as an intervetion target for treating pain and preventing a wide variety of diseases and conditions, for example, the treatment of pain including hyperalgesia, the use in eye disorders and eye pain, the treatment of pruritus caused by uremia and opioids, and the like.

Difelikefalin, a K-opioid receptor agonist that selectively targets peripheral K-opioid receptors to relieve pruritus and is used for postoperative analgesia, has already entered phase III clinical trials. Because Difelikefalin has a short half-life in vivo, patients require daily subcutaneous administration, resulting in poor patient compliance. The present disclosure is intended to provide a long-acting K-opioid receptor agonist, to reduce administration frequency, and to improve patient administration compliance.

### SUMMARY

The present disclosure provides a long-acting K-opioid receptor agonist and uses thereof.

To achieve the aforementioned purposes, the present disclosure first provides a compound as represented by structural formula I, as well as a pharmaceutically acceptable salt, a solvate, a chelate, or a non-covalent complex thereof, a prodrug based on the compound, or any mixture of the foregoing forms:

D-Phe-AA1-AA2-D-Lys-4-amino-4-piperidinecarboxylic acid-AA3-AA4(R)-AA5 structural formula I

AA1 in structural formula I is D-Tyr(Me), or D-Tyr(Et), or D-Tyr(Ipr), or D-Phe(2-F), or D-Phe(4-F), or D-Phe(2-Cl), or D-Phe(4-Cl), or D-Phe(2-Br), or D-Phe(4-Br), or D-Phe(2-I), or D-Phe(4-I), or D-Phe(4-Me), or D-Phe(4-Et), or D-Phe(4-Ipr), or D-Phe(4-NH₂), or D-Phe(4-NHCH₃), or D-Phe(4-NHCH₂CH₃), or D-Phe(4-NHCH(CH₃)₂), or D-Phe(4-N(CH₃)₂), or D-Phe(4-N(CH₃)CH₂CH₃);
AA2 in structural formula I is D-Leu, or D-cyclopropylalanine;
AA3 in structural formula I is (PEGₘ₁(CH₂)ₘ₂CO)ₘ₃-, or (AA6)ₘ₁, or is absent, wherein:
   m1 is an integer ranging from 1 to 10;
   m2 is an integer ranging from 1 to 5;
   m3 is an integer ranging from 1 to 5; and
AA6 is Gly, or D-Ala, or L-Ala, or D-Leu, or L-Leu, or D-Phe, or L-Phe, or D-Ser, or L-Ser, or D-Thr, or L-Thr, or D-Tyr, or L-Tyr, or D-Asp, or L-Asp, or D-Glu, or L-Glu, or D-Gln, or L-Gln, or D-Lys, or L-Lys, or D-Arg, or L-Arg, or D-His, or L-His;
AA4 in structural formula I is (AA7)ₙ, or is absent, wherein:
   n is an integer ranging from 1 to 10; and
   AA7 is D-Lys, or L-Lys, or D-Dap, or L-Dap, or D-Dab, or L-Dab, or D-Orn, or L-Orn, or D-Dah, or L-Dah, or D-Dao, or L-Dao;
AA5 in structural formula I is OH, or NH₂; and
R in structural formula I is HO₂C(CH₂)ₙ₁CO-(AA8)ₙ₂-(PEGₙ₃(CH₂)ₙ₄CO)ₙ₅-, or HO₂C(CH₂)ₙ₁CO-(AA8)ₙ₂-(AA9)ₙ₆-, wherein:
   n1 is an integer ranging from 10 to 20;
   n2 is an integer ranging from 1 to 5;
   n3 is an integer ranging from 1 to 30;
   n4 is an integer ranging from 1 to 5;
   n5 is an integer ranging from 1 to 5;
   n6 is an integer ranging from 1 to 10;
AA8 is yGlu, or εLys, or β-Ala, or γ-aminobutyric acid, or 5-Ava; and
AA9 is Ala, or Gly, or Leu, or Phe, or Ser, or Thr, or Tyr, or Asp, or Glu, or Gln, or Lys, or D-Lys, or Arg, or His.

The long acting K-opioid receptor agonist provided in the present disclosure includes a pharmaceutically acceptable salt, a solvate, a chelate, or a non-covalent complex thereof, a prodrug based on the compound, or any mixture of the foregoing forms.

The present disclosure also provides a pharmaceutical composition comprising a compound according to the present disclosure, and a use of the pharmaceutical composition in the manufacture of a medicament for treating a disease.

Furthermore, the pharmaceutical composition is used for relieving moderate to severe pruritus and for postoperative analgesia.

More contents involved in the present disclosure are described below in detail. Some of the contents can be comprehended by combining the examples of the present disclosure.

Unless otherwise indicated, all numbers expressing quantities of ingredients, reaction conditions, and the like used in the present disclosure are to be understood as being modified in all instances by the term "about" or "approximately". Accordingly, unless indicated to the contrary, the numerical parameters cited in the following description and the claims are approximations that may differ due to the difference in standard error found in their respective experimental conditions.

In the present disclosure, if there is a discrepancy or ambiguity between a chemical structure and a chemical name for a compound, the chemical structure shall prevail in defining the compound. The compounds described herein may contain one or more chiral centers and/or double bonds and the like, and may therefore exist as stereoisomers, including isomers of double bonds (such as geometric isomers), enantiomers, or diastereomers. Accordingly, any chemical structure within the scope of this disclosure, which contains such similar structures in part or in whole, is intended to encompass all possible enantiomers and diastereomers of the compound. This includes any single stereoisomer (such as a single geometric isomer, a single enantiomer, or a single diastereomer) as well as any mixture of these isomers. The mixtures of these racemates and stereoisomers can be further resolved into their constituent enantiomers or stereoisomers by a person skilled in the art using various separation techniques or methods of chiral synthesis.

The compounds as represented by structural formula I include, but are not limited to, optical isomers, racemates, and/or other mixtures of these compounds. In such cases, a single enantiomer or diastereomer, including an optically active isomer, may be obtained by asymmetric synthesis or by resolution of racemates. The resolution of racemates can be accomplished by various methods, such as conventional recrystallization using a resolving agent, or by chromatographic methods. Furthermore, the compounds as represented by structural formula I also include cis- and/or trans-isomers of double bonds.

The compounds of the present disclosure include, but are not limited to, the compounds as represented by structural formula I and all pharmaceutically acceptable forms thereof. Pharmaceutically acceptable forms of these compounds include various pharmaceutically acceptable salts, solvates, complexes, chelates, non-covalent complexes, prodrugs based on the above substances, and any mixtures of the foregoing forms.

### DETAILED DESCRIPTION

The present disclosure provides a long-acting K-opioid receptor agonist and uses thereof. A person skilled in the art can, by referring to the content of this disclosure, suitably modify the relevant parameters to practice the present disclosure. It is to be particularly noted that all similar substitutions and modifications obvious to a person skilled in the art are deemed to be included within the present disclosure. While the methods of the present disclosure have been described by preferred examples, it will be apparent to those skilled in the art that modifications, or suitable variations and combinations, can be made to the compounds and preparation methods described herein without departing from the content, spirit, and scope of the present disclosure to realize and apply the technology of the present disclosure. The full names corresponding to the English abbreviations used in the present disclosure are shown in the table below:

**Table 1**

| **English Abbreviations** | **Full Name** | **English Abbreviations** | **Full Name** |
|---|---|---|---|
| Fmoc | 9-fluorenylmethylox ycarbonyl | OtBu | tert-butoxy |
| tBu | tert-butyl | Boc | tert-butoxycarbonyl |
| Trt | trityl | Pbf | (2,3-dihydro-2,2,4,6,7-pentameth ylbenzofuran-5-yl)sulfonyl |
| Ala | alanine | Leu | leucine |
| Arg | arginine | Lys | lysine |
| Asn | asparagine | Phe | phenylalanine |
| Asp | aspartic acid | Pro | proline |
| Cys | cysteine | Ser | serine |
| Gln | glutamine | Thr | threonine |
| Glu | glutamic acid | Trp | tryptophan |
| Gly | glycine | Tyr | tyrosine |
| His | histidine | Val | valine |
| Ile | isoleucine | Dab | 2,4-diaminobutyric acid |
| 5-Ava | 5-aminovaleric acid | Orn | ornithine |
| Aib | aminoisobutyric acid | Dah | 2,7-diaminoheptanoic acid |
| Dap | 2,3-diaminopropionic acid | Dao | 2,8-diaminooctanoic acid |
| Ada | 2-aminoadipic acid | Apm | 2-aminopimelic acid |
| Asu | 2-aminosuberic acid | | |

### Example 1: preparation of compounds

The preparation method is solid-phase polypeptide synthesis, which comprises: preparing a peptide-resin by solid-phase polypeptide synthesis, cleaving the peptide-resin with an acid to obtain a crude product, and finally purifying the crude product to obtain a pure product. Wherein, the step of preparing the peptide-resin by solid-phase polypeptide synthesis involves sequentially coupling the corresponding protected amino acids of the following sequence onto a carrier resin via solid-phase coupling synthesis method to prepare the peptide-resin:
in the aforementioned preparation method, the Fmoc-protected amino acid is used at an amount of 1.2 to 6 times the total molar amount of the resin used, preferably 2.5 to 3.5 times.

In the aforementioned preparation method, the carrier resin has a substitution value of 0.3 to 1.5 mmol/g resin, preferably 0.6 to 1.0 mmol/g resin.

In a preferred embodiment of the disclosure, the solid-phase coupling synthesis method is as follows: the Fmoc protecting group is removed from the protected amino acid-resin obtained from the previous step, followed by a coupling reaction with the next protected amino acid. The deprotection step of removing the Fmoc group had a duration of 10 to 60 min, preferably 15 to 25 min. The coupling reaction had a duration of 60 to 300 min, preferably 100 to 140 min.

The coupling reaction requires the addition of a condensation reagent. The conde nsation reagent is selected from the group consisting of DIC (N,N-diisopropylcarbodiimid e), N,N-dicyclohexylcarbodiimide, benzotriazol-1-yl-oxy-tripyrrolidinophosphonium hexafluor ophosphate, 2-(7-aza-1H-benzotriazol-1-yl)-1,1,3,3-tetramethyluronium hexafluorophosphate, benzotriazol-N,N,N',N'-tetramethyluronium hexafluorophosphate, and O-(benzotriazol)-N,N,N', N'-tetramethyluronium tetrafluoroborate. The preferred one is N,N-diisopropylcarbodiimide. The condensation reagent is used at a molar amount of 1.2 to 6 times the total molar a mount of amino groups on the amino-resin, preferably 2.5 to 3.5 times.

The coupling reaction requires the addition of an activating agent. The activating agent is selected from the group consisting of 1-hydroxybenzotriazole and N-hydroxy-7-azabenzotriazole, preferably 1-hydroxybenzotriazole. The activating agent is used at an amount of 1.2 to 6 times the total molar amount of amino groups on the amino-resin, preferably 2.5 to 3.5 times.

In a preferred embodiment of the present disclosure, the reagent for removing the Fmoc protecting group is a mixed solution of PIP/DMF (piperidine/N,N-dimethylformamide), wherein the mixed solution contains 10% to 30% (V) piperidine. The amount of the Fmoc deprotection reagent used is 5 to 15 mL per gram of amino-resin, preferably 8 to 12 mL per gram of amino-resin.

More preferably, acidolysis agent used in the step of acidolysis of the peptide-resin is a mixed solvent of trifluoroacetic acid (TFA), 1,2-ethanedithiol (EDT), and water, wherein the mixed solvent has a volume ratio of: 80% to 95% TFA, 1% to 10% EDT, with the balance being water.

Even more preferably, the mixed solvent has a volume ratio of: 89% to 91% TFA, 4% to 6% EDT, with the balance being water. Most preferably, the mixed solvent has a volume ratio of: 90% TFA, 5% EDT, with the balance being water.

The acidolysis agent is used at an amount of 4 to 15 mL per gram of peptide-resin; preferably, 7 to 10 mL per gram of peptide-resin.

The cleavage step using the acidolysis agent has a duration of 1 to 6 h, preferably 3 to 4 h, at room temperature.

Furthermore, the crude product is purified by high-performance liquid chromatography and then lyophilized to obtain the pure product.

### 1. Synthesis of peptide-resin

Peptide-resin was prepared by taking a carrier resin and sequentially coupling the corresponding protected amino acids of the sequence via deprotection of Fmoc and coupling reactions:

### (1) Coupling of the first protected amino acid of the main chain

0.03 mol of the first protected amino acid and 0.03 mol of HOBt were dissolved in an appropriate amount of DMF. Separately, 0.03 mol of DIC was added slowly to the DMF solution of the protected amino acid with stirring. Reaction was performed at room temperature under stirring for 30 min to obtain a solution of activated protected amino acid, which was set aside for later use.

0.01 mol of Rink amide MBHA resin (substitution value was approximately 0.4 mmol/g) was taken, and deprotected by treatment with a 20% PIP/DMF solution for 25 min. The Fmoc-deprotected resin was obtained after washing and filtering.

The activated first protected amino acid solution was added to the Fmoc-deprotected resin, and the coupling reaction was carried out for 60 to 300 min. The resin containing one protected amino acid was obtained after filtering and washing.

### (2) Coupling of other protected amino acids of the main chain

Using the same method as for the coupling of the first amino acid of the main chain, the other corresponding protected amino acids of the main chain were sequentially coupled to obtain a resin containing the main chain amino acids.

### (3) Coupling of the first protected amino acid of the side chain

0.03 mol of the first protected amino acid of the side chain and 0.03 mol of HOBt were dissolved in an appropriate amount of DMF. Separately, 0.03 mol of DIC was added slowly to the DMF solution of the protected amino acid with stirring. Reaction was performed at room temperature under stirring for 30 min to obtain a solution of activated protected amino acid.

2.5 mmol of tetrakis(triphenylphosphine)palladium(0) and 25 mmol of phenylsilane were dissolved in an appropriate amount of dichloromethane, and a deprotection reaction was carried out for 4 h. The Alloc-deprotected resin was obtained after filtering and washing, which was set aside for later use.

The solution of the activated first protected amino acid of the side-chain was added to the Alloc-deprotected resin, and the coupling reaction was carried out for 60 to 300 min. The resin containing the first protected amino acid of the side chain was obtained after filtering and washing.

### (4) Coupling of other protected amino acids of the side chain

Using the same method as for the coupling of the first protected amino acid of the main chain, the corresponding protected amino acids and mono-protected fatty acids of the side chain were sequentially coupled to obtain the peptide-resin.

### 2. Preparation of the crude product

The peptide-resin obtained above was treated with a cleavage reagent (10 mL of cleavage reagent per gram of resin) with a volume ratio of TFA:water:EDT = 95:5:5. The mixture was stirred evenly and allowed to react at room temperature for 3 h. The reaction mixture was filtered through a fritted funnel, and the filtrate was collected. The resin was washed three times with a small amount of TFA. The filtrates were combined and concentrated under reduced pressure. Anhydrous diethyl ether was added and precipitate was produced. The precipitate was washed three times with anhydrous diethyl ether and then dried under vacuum to yield an off-white powder.

### 3. Preparation of the pure product

The crude product concentrate obtained above was purified by filtering through a 0.45 µm mixed microporous filter membrane and set aside for later use.

Purification was performed using high-performance liquid chromatography. The chromatographic packing material for purification was 10 µm reverse-phase C18 material, and the mobile phase system consisted of 0.1% TFA/water and 0.1% TFA/acetonitrile. For a 30 mm*250 mm chromatography column, the flow rate was 20 mL/min. A gradient elution and cyclic loading purification was used. The crude product solution was loaded onto the column, and the mobile phase was initiated for elution. The main peak was collected, and after removing acetonitrile by evaporation, a purified intermediate concentrate was obtained.

The purified intermediate concentrate was filtered through a 0.45 µm membrane and set aside for later use. Salt exchange was performed using high-performance liquid chromatography. The mobile phase system was 1% acetic acid/water-acetonitrile, and the chromatographic packing material for purification was 10 µm reverse-phase C18 material. For a 30 mm*250 mm chromatography column, the flow rate was 20 mL/min (the flow rate can be adjusted according to different chromatography column specifications). A gradient elution and cyclic loading method was used. The sample was loaded onto the column, the mobile phase was initiated for elution, and chromatograms were collected. Changes in absorbance were observed, and the main peak from the salt exchange was collected. The purity was checked by analytical liquid chromatography. The fractions containing the main peak from the salt exchange were combined, concentrated under reduced pressure to obtain a purified product aqueous solution in acetic acid, and then lyophilized to obtain the pure product.

Using the method described above, the following compounds were synthesized:

**Table 2**

| | |
|---|---|
| Compound 1 | D-Phe-D-Tyr(Et)-D-Leu-D-Lys-4-aminopiperidine-4-carboxamide-Lys (PEG₄CH₂CO-yGlu-octadecanedioic acid)-NH₂ |
| Compound 2 | D-Phe-D-Tyr(Et)-D-Leu-D-Lys-4-aminopiperidine-4-carboxamide-PEG ₁CH₂CO-Lys(PEG₄CH₂CO-yGlu-octadecanedioic acid)-NH₂ |
| Compound 3 | D-Phe-D-Tyr(Et)-D-Leu-D-Lys-4-aminopiperidine-4-carboxamide-AEE A-Lys(PEG₄CH₂CO-yGlu-octadecanedioic acid)-NH₂ |
| Compound 4 | D-Phe-D-Tyr(Et)-D-Leu-D-Lys-4-aminopiperidine-4-carboxamide-PEG ₄CH₂CO-Lys(PEG₄CH₂CO-yGlu-octadecanedioic acid)-NH₂ |
| Compound 5 | D-Phe-D-Tyr(Me)-D-Leu-D-Lys-4-aminopiperidine-4-carboxamide-AE EA-Lys(PEG₄CH₂CO-γGlu-octadecanedioic acid)-NH₂ |
| Compound 6 | D-Phe-D-Phe(4-Cl)-D-Leu-D-Lys-4-aminopiperidine-4-carboxamide-A EEA-Lys(PEG₄CH₂CO-γGlu-octadecanedioic acid)-NH₂ |
| Compound 7 | D-Phe-D-Phe(4-F)-D-Leu-D-Lys-4-aminopiperidine-4-carboxamide-AE EA-Lys(PEG₄CH₂CO-γGlu-octadecanedioic acid)-NH₂ |
| Compound 8 | D-Phe-D-Tyr(Et)-D-Leu-D-Lys-4-aminopiperidine-4-carboxamide-AEE A-Lys(PEG₃CH₂CO-γGlu-octadecanedioic acid)-NH₂ |
| Compound 9 | D-Phe-D-Tyr(Et)-D-Leu-D-Lys-4-aminopiperidine-4-carboxamide-AEE A-Lys(PEG₃CH₂CO-γGlu-octadecanedioic acid)-NH₂ |
| Compound 10 | D-Phe-D-Tyr(Et)-D-Leu-D-Lys-4-aminopiperidine-4-carboxamide-Gly-Lys(PEG₄CH₂CO-γGlu-octadecanedioic acid)-NH₂ |
| Compound 11 | D-Phe-D-Tyr(Et)-D-Leu-D-Lys-4-aminopiperidine-4-carboxamide-Gly-Gly-Lys(PEG₄CH₂CO-γGlu-octadecanedioic acid)-NH₂ |
| Compound 12 | D-Phe-D-Tyr(Et)-D-Cyclopropylalanine-D-Lys-4-aminopiperidine-4-car boxamide-AEEA-Lys(PEG₄CH₂CO-γGlu-octadecanedioic acid)-NH₂ |

### Example 2: determination of agonist activity

### 1. Determination Method

Gi-coupled human κ opioid receptor (OPRK1), upon stimulation with its specific opioid receptor agonists, can inhibit the intracellular adenylate cyclase pathway, and decrease cAMP levels. Forskolin can stimulate cAMP release in cell lines highly expressing opioid receptors. By stimulating cell lines stably transfected with opioid receptors, the inhibitory effect of a test compound on forskolin-stimulated cAMP release can be measured to determine the activity of the compound. The relative light units (RLU) from the cells after stimulation with different doses were measured via the homogeneous time-resolved fluorescence (HTRF) technique, and the EC50 of the agonist was subsequently calculated. This activity determination method is a currently commonly used method for determining the activities of opioid receptor agonists at home and abroad.

A CHO-K1 cell line stably expressing opioid receptors was used. Stably transfected cells were stimulated with various concentrations of agonists (under the stimulation of a certain concentration of forskolin). The relative light units from the cells after stimulation with different doses were measured, from which the EC50 value of the agonist was subsequently obtained by calculation.

### 2. Results of determination

The results of the determination are presented in the table below:

**Table 3**

| **Compounds** | **Result of Activity Determination** | |
|---|---|---|
| | **EC50 (pmol)** | **Relative Activity%** |
| Difelikefalin | 652.6 | 100% |
| Compound 1 | 4628.4 | 15.6% |
| Compound 2 | 3471.3 | 20.7% |
| Compound 3 | 3021.3 | 23.8% |
| Compound 4 | 4183.3 | 17.2% |
| Compound 5 | 5305.7 | 13.6% |
| Compound 6 | 5879.3 | 12.2% |
| Compound 7 | 6869.5 | 10.5% |
| Compound 8 | 4078.8 | 17.7% |
| Compound 9 | 4379.9 | 16.4% |
| Compound 10 | 4763.5 | 15.1% |
| Compound 11 | 4265.4 | 16.9% |
| Compound 12 | 4729.0 | 15.2% |

### Example 3: determination of preliminary pharmacokinetic properties

The compound with the best activity was selected for determination of its preliminary pharmacokinetic properties. Two male Macaca fascicularis were used as experimental animals. A subcutaneous administration was performed at a dose of 0.1 mg/kg. Venous blood samples were collected before administration (0 h) and at 1 h, 2 h, 3 h, 4 h, 8 h, 12 h, 18 h, 24 h, 48 h, 96 h, 144 h, and 168 h post-administration. Plasma samples were separated by centrifugation. The plasma concentration of the compound in the plasma samples was determined using liquid chromatography-mass spectrometry. The half-life of the compound following subcutaneous (SC) administration was shown in the table below:

**Table 4**

| Compound | t1/2 (h) |
|---|---|
| Compound 3 | 55.1 |

The foregoing are merely preferred embodiments of the present disclosure. It should be pointed out that for a person of ordinary skill in the art, various improvements and modifications can be made without departing from the principles of the present disclosure, and these improvements and modifications should also be considered as falling within the scope of protection of the present disclosure.

## Claims

1. A long acting K-opioid receptor agonist having a structure of structural formula I:
D-Phe-AA1-AA2-D-Lys-4-aminopiperidine-4-carboxylic acid-AA3-AA4(R)-AA5 structural formula I
AA1 in structural formula I is D-Tyr(Me), or D-Tyr(Et), or D-Tyr(Ipr), or D-Phe(2-F), or D-Phe(4-F), or D-Phe(2-Cl), or D-Phe(4-Cl), or D-Phe(2-Br), or D-Phe(4-Br), or D-Phe(2-I), or D-Phe(4-I), or D-Phe(4-Me), or D-Phe(4-Et), or D-Phe(4-Ipr), or D-Phe(4-NH₂), or D-Phe(4-NHCH₃), or D-Phe(4-NHCH₂CH₃), or D-Phe(4-NHCH(CH₃)₂), or D-Phe(4-N(CH₃)₂), or D-Phe(4-N(CH₃)CH₂CH₃);
AA2 in structural formula I is D-Leu, or D-cyclopropylalanine;
AA3 in structural formula I is (PEGₘ₁(CH₂)ₘ₂CO)ₘ₃-, or (AA6)ₘ₁, or is absent, wherein:
m1 is an integer ranging from 1 to 10;
m2 is an integer ranging from 1 to 5;
m3 is an integer ranging from 1 to 5; and
AA6 is Gly, or D-Ala, or L-Ala, or D-Leu, or L-Leu, or D-Phe, or L-Phe, or D-Ser, or L-Ser, or D-Thr, or L-Thr, or D-Tyr, or L-Tyr, or D-Asp, or L-Asp, or D-Glu, or L-Glu, or D-Gln, or L-Gln, or D-Lys, or L-Lys, or D-Arg, or L-Arg, or D-His, or L-His;
AA4 in structural formula I is (AA7)ₙ, or is absent, wherein:
n is an integer ranging from 1 to 10; and
AA7 is D-Lys, or L-Lys, or D-Dap, or L-Dap, or D-Dab, or L-Dab, or D-Orn, or L-Orn, or D-Dah, or L-Dah, or D-Dao, or L-Dao;
AA5 in structural formula I is OH, or NH₂; and
R in structural formula I is HO₂C(CH₂)ₙ₁CO-(AA8)ₙ₂-(PEGₙ₃(CH₂)ₙ₄CO)ₙ₅-, or HO₂C(CH₂)ₙ₁CO-(AA8)ₙ₂-(AA9)ₙ₆-, wherein:
n1 is an integer ranging from 10 to 20;
n2 is an integer ranging from 1 to 5;
n3 is an integer ranging from 1 to 30;
n4 is an integer ranging from 1 to 5;
n5 is an integer ranging from 1 to 5;
n6 is an integer ranging from 1 to 10;
AA8 is yGlu, or εLys, or β-Ala, or γ-aminobutyric acid, or 5-Ava; and
AA9 is Ala, or Gly, or Leu, or Phe, or Ser, or Thr, or Tyr, or Asp, or Glu, or Gln, or Lys, or D-Lys, or Arg, or His.

2. The long acting K-opioid receptor agonist according to claim 1, which includes a pharmaceutically acceptable salt, a solvate, a chelate, or a non-covalent complex thereof, a prodrug based on the compound, or any mixture of the foregoing forms.

3. The long acting K-opioid receptor agonist according to claim 1 or 2, for use in the manufacture of a pharmaceutical composition for treating a disease.

4. The long acting K-opioid receptor agonist according to claim 3, wherein the pharmaceutical composition is used for relieving moderate to severe pruritus in a patient and for postoperative analgesia.

5. A pharmaceutical composition, comprising the long acting K-opioid receptor agonist according to any one of claims 1 to 4.

6. Use of the long acting K-opioid receptor agonist according to any one of claims 1 to 4 or the pharmaceutical composition according to claim 5 in the manufacture of a medicament for relieving moderate to severe pruritus in a patient and for postoperative analgesia.
